# EUROPÄISCHE PATENTSCHRIFT

(11) **EP 2 316 408 B1**
(45) Veröffentlichungstag und Bekanntmachung des Hinweises auf die Patenterteilung: **03.07.2013**
(21) Anmeldenummer: 10188764.4
(22) Anmeldetag: 25.10.2010
(51) Int. Cl.: A61K 8/06, A61K 8/34, A61K 8/37, A61Q 13/00

(54) **Ethanolfreie Parfümölmikroemulsion**
Ethanol-free perfume oil microemulsion
Microémulsion de parfum sans éthanol

(43) Veröffentlichungstag der Anmeldung: 04.05.2011
(73) Patentinhaber: Symrise AG, 37603 Holzminden (DE)
(72) Erfinder: Wiedemann, Jörn, 37603, Holzminden (DE); Kaufhold, Astrid, 37603 Holzminden (DE)
(74) Vertreter: Eisenführ, Speiser & Partner

(56) Entgegenhaltungen:
- EP-A2- 1 027 880
- WO-A1-2005/123028
- US-A1- 2009 165 360
- US-A1- 2009 238 787

## Beschreibung

Die vorliegende Erfndung betrifft ethanolfreie Parfümölmikroemulsionen, umfassend oder bestehend aus (a) Wasser, (b) einem oder mehreren vicinalen Diol(en), (c) einem, zwei oder drei Lösungsmitteln zur Reduktion der Klebrigkeit, ausgewählt aus der Gruppe bestehend aus Glycerol, Isobuttersäure-1-hydroxy-2,2,4-trimethyl-3-pentylester (Verbindung der Formel (1)) und Isobuttersäure-3-hydroxy-2,2,4-trimethyl-1-pentylester (Verbindung der Formel (2)), sowie (d) einem oder mehreren Riechstoff(en) und (e) einer oder mehreren weiteren Substanzen, wobei, bezogen auf das Gesamtgewicht der Parfümölmikroemulsion, die Gesamtmenge an Lösungsmitteln (c) in der Parfümölmikroemulsion 1 bis 20 Gew.-%, bevorzugt 2 bis 10 Gew.-%, besonders bevorzugt 3 bis 5 Gew.-%, 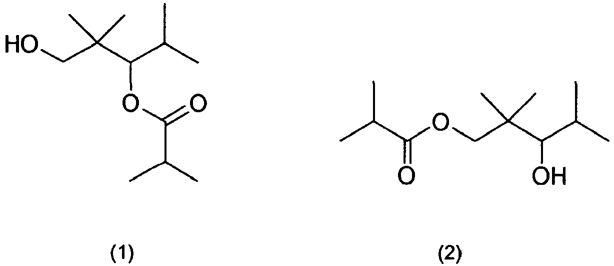 beträgt.

Zudem betrifft die vorliegende Erfindung die Verwendung von Glycerol, der Verbindung der Formel (1), der Verbindung der Formel (2) oder einer Mischung umfassend oder bestehend aus Glycerol, einer Verbindung der Formal (1) und/oder einer Verbindung der Formel (2) zur Reduzierung der Klebrigkeit einer ethanolfreien Parfümölmikroemulsion sowie ein Verfahren zum Reduzieren der Klebrigkeit einer ethanolfreien Parfümölmikroemulsion.

Des Weiteren betrifft die vorliegende Erfindung Artikel, die eine erfindungsgemäße ethanolfreie Parfümölmikroemulsion umfassen.

Weitere Aspekte der vorliegenden Erfindung ergeben sich aus der folgenden Beschreibung, den Beispielen sowie den beigefügten Patentansprüchen.

In der Parfümindustrie besteht seit längerem ein Bedarf nach wässrigen, ethanolfreien Parfümölzusammensetzungen. Zwar sind Ethanol enthaltende Lösungen von Parfümölen weit verbreitet, jedoch weisen solche Zusammensetzungen diverse, teilweise für den Konsumenten nicht akzeptable Nachteile auf. Gründe, die gegen den Einsatz von Ethanol in Parfümölzusammensetzungen sprechen, beruhen beispielsweise auf einem dem Ethanol zuzuordnenden Risiko von Hautirritationen, religiösen Hintergründen oder dem krebserregende Potential von Ethanol sowie gesetzlichen Regelungen zur Vermeidung von flüchtigen organischen Stoffen (VOCs). Dem steht die Akzeptanz von wässrigen Formulierungen gegenüber, die unbrennbar sind und von den Konsumenten generell als ungefährlich eingeschätzt werden. Dementsprechend hat die Parfümindustrie bereits in der Vergangenheit versucht, geeignete, ethanolfreie Parfümölzusammensetzungen zu entwickeln. Bisher gelang es jedoch nicht, ethanolfreie Parfümölzusammensetzungen bereitzustellen, die von den Konsumenten (uneingeschränkt) akzeptiert worden sind.

Im Stand der Technik bekannte ethanolfreie Parfümölzusammensetzungen enthalten in der Regel neben Parfümöl(en), Wasser und gegebenenfalls Konservierungsmittel(n) ein oder mehrere Tenside, welche zur Solubilisierung des bzw. der Parfümöle eingesetzt werden. Tenside können jedoch Hautirritationen hervorrufen und führen in der Regel dazu, dass sich die Parfümzusammensetzung aus Sicht der Konsumenten zu klebrig anfühlt.

In US 2003/0186836 A1 werden alkoholfreie, wässrige Parfümölzusammensetzungen beschrieben, welche (anstelle von Ethanol) Isoprenglycol (3-Methyl-1,3-Butandiol) als Lösungsmittel enthalten. Die in US 2003/0186836 A1 beschriebenen Parfümölzusammensetzungen enthalten jedoch zudem ein oder mehrere Tenside, so dass diese aufgrund der daraus resultierenden Klebrigkeit von den Konsumenten abgelehnt werden.

In WO 2005/123028 A1 wird eine ethanolfreie, parfümierte, wässrige Mikroemulsion beschrieben, die als Lösungsmittel ein vicinales Diol umfasst. Zwar sind Tenside gemäß WO 2005/123028 A1 nur optional in der Mikroemulsion enthalten. Jedoch hat sich herausgestellt, dass die in WO 2005/123028 A1 beschriebenen Mikroemulsionen sich aus Sicht der Konsumenten dennoch aufgrund der enthaltenen Diole zu klebrig anfühlen.

So besteht auch weiterhin das Interesse, ethanolfreie Parfümölzusammensetzungen mit reduzierter Klebrigkeit bereitzustellen. Zudem sollten dabei vorzugsweise ausschließlich Stoffe eingesetzt werden, die auf dem Markt für die entsprechende Anwendung zugelassen sind. Des Weiteren ist es erstrebenswert, dass solche Parfümölzusammensetzungen transparent sind, um vielfältige Einsatzmöglichkeiten in der Parfümindustrie zu gewährleisten.

Primäre Aufgabe der vorliegenden Erfindung war es daher, eine ethanolfreie Parfümölzusammensetzung bereitzustellen, die eine reduzierte oder von den Konsumenten nicht bzw. nicht als störend wahrgenommene Klebrigkeit besitzt und vorzugsweise den vorangehend erwähnten Anforderungen genügt.

Eine weitere Aufgabe der vorliegenden Erfindung bestand darin, Verwendungen und Verfahren bereitzustellen, wodurch sich die Klebrigkeit von ethanolfreien Parfümölzusammensetzungen reduzieren lässt.

Des Weiteren bestand eine Aufgabe der vorliegenden Erfindung darin, Artikel bereitzustellen, die eine vorangehend beschriebene ethanolfreie Parfümölzusammensetzung mit reduzierter Klebrigkeit enthalten.

Weitere der vorliegenden Erfindung zugrunde liegende Aufgaben ergeben sich aus der vorliegenden Beschreibung, den Beispielen sowie insbesondere den beigefügten Patentansprüchen.

Die primäre Aufgabe der vorliegenden Erfindung wird gelöst durch eine ethanolfreie Parfümölmikroemulsion, umfassend oder bestehend aus:
(a) Wasser,
(b) einem oder mehreren vicinalen Diol(en),
(c) einem, zwei oder drei Lösungsmittel(n) zur Reduktion der Klebrigkeit, ausgewählt aus der Gruppe bestehend aus Glycerol, Isobuttersäure-1-hydroxy-2,2,4-trimethyl-3-pentylester (Verbindung der Formel (1)) und lsobuttersäure-3-hydroxy-2,2,4-trimethyl-1-pentylester (Verbindung der Formel (2)),
(d) einem oder mehreren Riechstoff(en), und
(e) optional einer oder mehreren weiteren Substanzen, wobei, bezogen auf das Gesamtgewicht der Parfümölmikroemulsion, die Gesamtmenge an Lösungsmittel (c) in der Parfümölmikroemulsion 1 bis 20 Gew.-%, bevorzugt 2 bis 10 Gew.-%, besonders bevorzugt 3 bis 5 Gew.-%, beträgt.

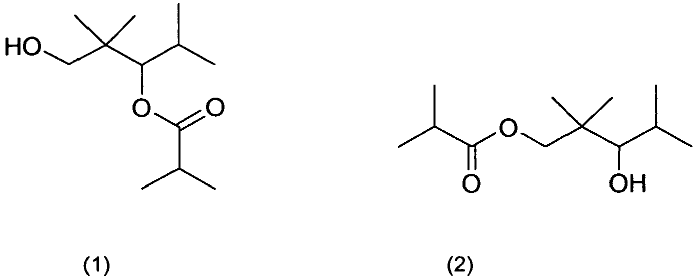

Für die Zwecke der vorliegenden Erfindung ist der bzw. sind die Bestandteile (d) nicht ausgewählt aus der Gruppe bestehend aus Glycerol, Isobuttersäure-l-hydroxy-2,2,4-trimethyl-3-pentylester (Verbindung der Formel (1)) und Isobuttersäure-3-hydroxy-2,2,4-trimethyl-1-pentylester (Verbindung der Formel (2)).

Wie oben erwähnt, betrifft die vorliegende Erfindung ethanolfreie Parfümölmikroemulsionen. Unter einer "ethanolfreien" Parfümölmikroemulsion ist im Rahmen der vorliegenden Erfindung eine vollständig oder zumindest im wesentlichen ethanolfreie Parfümölmikroemulsion zu verstehen. Bevorzugt umfasst eine erfindungsgemäße Parfümölmikroemulsion demnach keinen Ethanol oder weniger als 0,01 Gew.-% Ethanol, vorzugsweise weniger als 0,001 Gew.-% Ethanol, bezogen auf das Gesamtgewicht der Parfümölmikroemulsion.

Der Begriff "Mikroemulsion" bezeichnet im Rahmen der vorliegenden Erfindung eine makroskopisch homogene, optisch transparente und thermodynamisch stabile Mischung, die
- mindestens eine hydrophile,
- mindestens eine lipophile und
- mindestens eine amphiphile Substanz
umfasst.

Eine Mikroemulsion umfasst eine hydrophile Phase, eine lipophile Phase und eine amphiphile Phase. In einer erfindungsgemäßen Parfümölmikroemulsion enthält die hydrophile Phase vorzugsweise Bestandteil (a), d.h. Wasser, sowie gegebenenfalls Glycerol (Bestandteil (c)) und optional ein oder mehrere weitere Substanzen gemäß Bestandteil (e), z.B. Konservierungsmittel, Farbstoffe und/oder hydrophobe Lichtschutzmittel. Die lipohile Phase umfasst vorzugsweise Bestandteil (d), z.B. in Form eines Parfümöls, bzw. einen oder mehrere der Riechstoffe gemäß Bestandteil (d) sowie gegebenenfalls Isobuttersäure-1-hydroxy-2,2,4-trimethyl-3-pentylester (Verbindung der Formel (1)) und/oder Isobuttersäure-3-hydroxy-2,2,4-trimethyl-1-pentylester (Verbindung der Formel (2)) (Bestandteil (c)), und optional eine oder mehrere Substanzen gemäß Bestandteil (e), z.B. Konservierungsmittel, lipophile Lichtschutzmittel, Farbstoffe und/oder Stabilisatoren, insbesondere Radikalfänger. Die amphiphile Phase umfasst vorzugsweise ein oder mehrere vicinale Diole gemäß Bestandteil (b) sowie gegebenenfalls einen oder mehrere Bestandteile eines enthaltenen Parfümöls. Je nach enthaltenen Substanzen bzw. Bestandteilen in einer erfindungsgemäßen Parfümölmikroemulsion können sich die vorstehend genannten Substanzen gegebenenfalls auch in einer anderen Phase als der oben genannten anordnen bzw. befinden, z.B. ist es möglich, dass ein oder mehrere der vicinalen Diole gemäß Bestandteil (b) sich in der hydrophilen oder lipophilen Phase befinden..

Ein "Parfümöl" (allein) umfasst für die Zwecke der vorliegenden Erfindung vorzugsweise weniger als 10 Gew.-% Trägerstoffe, vorzugsweise keine Trägerstoffe, insbesondere kein Dipropylenglycol, Diethylphthalat, Triethylcitrat oder Isopropylmyristat, sondern besteht im Wesentlichen aus Riechstoffen. Demnach ist eine erfindungsgemäße Parfümölmikroemulsion vorzugsweise (im Wesentlichen) frei von (solchen) Trägerstoffen.
Mit Parfümölen werden insbesondere Parfüme hergestellt, indem man sie in Lösungen gibt, die beim Verdampfen die Riechstoffe "mitreißen" und so dem Riechorgan des Anwenders, d. h. des Menschen, den Sinneseindruck eines bestimmten Geruchs vermitteln. Neben dem Einsatz in Parfümen, Eau des Parfums, oder Eau de Toilettes dienen Parfümöle häufig auch der Erzeugung eines bestimmten Duftes in Wohnräumen, wie beispielsweise bei der Anwendung in Duftlampen, Zerstäubern oder Diffusern. Darüber hinaus können Parfümöle auch in unzähligen weiteren Artikeln bzw. Zubereitungen eingesetzt werden, beispielsweise von Schuhcremes bis Haarshampoos, in WC-Reinigern, Waschpulvern oder Katzensteinen.

Es war besonders überraschend, dass sich durch die Bestandteile (c) einer erfindungsgemäßen Parfümölmikroemulsion vorteilhafterweise die Klebrigkeit ethanolfreier Parfümölmikroemulsionen besonders gut reduzieren lässt (siehe hierzu unten, Testbeispiel).

Ein weiterer Vorteil erfindungsgemäßer Parfümölmikroemulsionen besteht darin, dass diese gegenüber Ethanol-haltigen sowie zum Teil auch im Stand der Technik bekannten, ethanolfreien Parfümölzusammensetzungen bei den Konsumenten ein reduziertes Gefühl der Austrocknung auf der Haut hervorrufen (siehe hierzu unten, Testbeispiel).

Vorteilhafterweise ist eine erfindungsgemäße Parfümölmikroemulsion auch thermodynamisch stabil und trennt sich bei Temperaturen, die gewöhnlich in Haushalten auftreten, vorzugsweise bei Temperaturen im Bereich von 0 bis 40 °C, nicht.

Des Weiteren führen die Bestandteile (b) und (c), insbesondere die Lösungsmittel (c), vorteilhafterweise nicht zu einer Trübung der Parfümölmikroemulsion. Zudem sind die Lösungsmittel (c) vorteilhafterweise für die Verwendung in kosmetischen Mitteln bzw. Zubereitungen zugelassen.

Wie vorangehend erwähnt, sind die Lösungsmittel (c) vorteilhafterweise geeignet, die Klebrigkeit ethanolfreier Parfümölmikroemulsionen zu reduzieren. Neben den Lösungsmitteln (c) kann eine erfindungsgemäße Parfümölmikroemulsion - als Bestandteil(e) (e)-noch weitere Substanzen zur Reduktion der Klebrigkeit enthalten. Besonders bevorzugt ist jedoch eine erfindungsgemäße ethanolfreie Parfümölmikroemulsion wie vorangehend beschrieben, wobei die Parfümölmikroemulsion eine Gesamtmenge an Lösungsmitteln (c) umfasst, die ausreicht, um im Vergleich zu einer identischen Parfümölmikroemulsion ohne Lösungsmittel (c) die Klebrigkeit der Parfümölmikroemulsion (signifikant) zu reduzieren.

Im Rahmen der vorliegenden Erfindung werden als vicinale Diole (b) vorzugsweise lineare, vicinale Diole eingesetzt, insbesondere vicinale Diole mit fünf bis acht Kohlenstoff-Atomen. Besonders bevorzugt ist bzw. sind das bzw. eines, mehrere oder sämtliche der vicinalen Diole (b) ausgewählt aus der Gruppe bestehend aus 2-Pentandiol, 1,2-Hexandiol, 1,2-Heptandiol und 1,2 Oktandiol. Die vicinalen Diole (b) können entweder einzeln oder in beliebiger Kombination miteinander eingesetzt werden.

Besonders bevorzugt ist eine ethanolfreie Parfümölmikroemulsion wie vorangehend beschrieben, wobei das bzw. eines der vicinalen Diole (b) 1,2-Hexandiol ist. Insbesondere für den Fall, dass eine erfindungsgemäße Parfümölmikroemulsion genau ein vicinales Diol enthält, ist dieses vorzugsweise 1,2-Hexandiol.

Sofern eine erfindungsgemäße Parfümölmikroemulsion zwei oder mehrere vicinale Diole (b) umfasst, werden vorzugsweise 1,2-Pentandiol und 1,2-Oktandiol eingesetzt. Besonders bevorzugt sind erfindungsgemäße Parfümölmikroemulsionen umfassend 1,2-Pentandiol und 1,2-Oktandiol sowie gegebenenfalls ein oder mehrere weitere Diole, wobei das Verhältnis der Gesamtmenge an 1,2-Pentandiol zu 1,2-Oktandiol in der Parfümölmikroemulsion, bezogen auf das Gewicht, vorzugsweise im Bereich von 6 zu 1 bis 2 zu 1, bevorzugt im Bereich von 5 zu 1 bis 3 zu 1, liegt und besonders bevorzugt etwa 4 zu 1 beträgt.

Wie sich im Rahmen eigener Untersuchungen herausgestellt hat, sind die Verbindungen der Formeln (1) und (2), wie vorangehend beschrieben, für die Zwecke der vorliegenden Erfindung besonders bevorzugt. Durch den Einsatz dieser Verbindungen lässt sich die Klebrigkeit von ethanolfreien Parfümölmikroemulsionen, insbesondere in Kombination mit vorangehend beschriebenen, erfindungsgemäß einzusetzenden Diolen (b), besonders gut reduzieren (siehe hierzu unten, Testbeispiel). Zudem besitzen die Verbindungen der Formeln (1) und (2) vorteilhafterweise keinen bzw. einen nur sehr schwachen Eigengeruch, wodurch sie für die Zwecke der vorliegenden Erfindung besonders geeignet sind.

Dementsprechend ist eine erfindungsgemäße ethanolfreie Parfümölmikroemulsion besonders bevorzugt, wobei die bzw. zwei der Lösungsmittel (c) Isobuttersäure-1-hydroxy-2,2,4-trimethyl-3-pentylester (Verbindung der Formel (1)) und Isobuttersäure-3-hydroxy-2,2,4-trimethyl-1-pentylester (Verbindung der Formel (2)) sind. Weiter bevorzugt beträgt in einer solchen Parfümölmikroemulsion das Verhältnis der Gesamtmenge an Isobuttersäure-1-hydroxy-2,2,4-trimethyl-3-pentylester (Verbindung der Formel (1)) zu Isobuttersäure-3-hydroxy-2,2,4-trimethyl-1-pentylester (Verbindung der Formel (2)) in der Parfümölmikroemulsion, bezogen auf das Gewicht, 99 : 1 bis 1 : 99, vorzugsweise 70 : 30 bis 30 : 70, vorzugsweise im Bereich von 60 : 40 bis 40 : 60, besonders bevorzugt etwa 44 : 56.

Der bzw. einer, mehrere oder sämtliche Riechstoffe (b) einer erfindungsgemäßen ethanolfreien Parfümölmikroemulsion ist bzw. sind vorzugsweise ausgewählt aus der Gruppe bestehend aus: 1-Phenyl-2-methyl-2-propylacetat, 2-Methylbutylbutyrat, Aldron (4-[(3,3-Dimethylbicyclo[2.2.1]hept-2-yl)methyl]-2-methylcyclohexanon), Allyl-2-cyclohexyloxyglycolat, Allyl-2-pentyloxyglycolat, Allyl-3-cyclohexylpropionat, Allylcapronat, Amarocit (1,1-Dimethoxy-2,2,5-trimethyl-4-hexen), Ambral (Dodecahydro-3,8,8,11a-tetramethyl-5H-3.5a-epoxynaphth[2.1-c]oxepin), Ambrettolid (9-Hexadecen-16-olid), Ambrinol S (1,2,3,4,4a,5,6,7-Octahydro-2,6,6-trimethyl-2-napthalinol), Ambrinolepoxid, Ambrocenide (4aR,5R,7aS,9R)-Octahydro-2,2,5,8,8,9a-hexamethyl-4H-4a,9-Methanoazuleno(5,6-d)-1,3-dioxol), Ambroxid (3a,6,6,9a-Tetramethyldodecahydronaphtho[2,1-b]-furan), Amylformiat, Aurelione (7-Cyclohexadecen-1-on und 8-Cyclohexadecen-1-on), Boronal [2-Methyl-4-(2,6,6-trimethyl-1-cyclohexen-1-yl)-butenal], Brahmanol [2-Methyl-4-(2,2,3-trimethyl-3-cyclopentenyl)-butanol], Buccoxime (1,5-Dimethylbicyclo[3.2.1]octan-8-onoxim), Butylacetat, Cantryl (2,2,3-Trimethyl-3-cyclopentenyl-1-acetonitril), Cassix 150 (4-Methoxy-2-methyl-2-butanthiol), Chrysantheme [1-(2,4-Dimethyl-3-cyclohexen-1-yl)-2,2-dimethyl-1-propanon], cis-3-Hexenylacetat, Citronellylbutyrat, Citronellyltiglinat (3,7-Dimethyl-6-octenyl-2-methylcrotonat), Citronitril (3-Methyl-5-phenyl-2-pentennitril), Citrowanil B (alpha-Ethenylalpha-methyl-benzolproprannitril), Claritone (2,4,7-Tetramethyl-6-octen-3-on), Corps Racine VS [2-(3-Phenylpropyl)pyridin], Coumarone (1-(2-Benzofuranyl)-ethanon), Cyclogalbanat (Allylcyclohexyloxyacetat), Cyclohexylmagnol (alpha-Methylcyclohexanpropanol), Datilat (1-Cyclohexylethylcrotonat), Ethyl-2-methylbutyrat, Ethylisobutyrat, Ethylisovalerat, Ethyltricyclo[5.2.1.0^{2,6}]decan-2-ylcarboxylat, Farenal (2,6,10-Trimethyl-9-undecenal), Filbertone (5-Methyl-2-hepten-4-on), Fleursandol (4-(3a,4,5,6,7,7a-Hexahydro-4,7-methano-1H-inden-6-yl)-3-methyl-3-buten-2-ol), Florazon (4-Ethyl-alpha,alpha-dimethyl benzolpropanal), Floropal (2,4,6-Trimethyl-4-phenyl-1,3-dioxan), Fragolane [(2,4-Dimethyl-[1,3]dioxolan-2-yl)essigsäureethylester)], Frutinat (But-2-ensäure-1,3-dimethylbutylester), gamma-Decalactone, Geranylacetat, Geranylbutyrat, Geranyltiglinat (trans-3,7-Dimethyl-2,6-octadienyl-2-methylcrotonat), Globalide [(11/12)-Pentadecen-15-olid], Globanone (8-Cyclohexadecen-1-on), Hexylbutyrat, Hydrocitronitril (beta-Methyl-benzolpentannitril), Indianol (4-[3a,4,5,6,7,7a-Hexahydro-4,7-methano-1H-inden-5(6)-yl]-3-methyl-3-buten-2-ol), Indoflor (4,4a,5,9b-Tetrahydoindeno[1,2-d]-m-dioxin), Irisnitril (2-Nonenylnitril), Isoamylacetat, Isoamylisovalerianat, Isodamascon [1-(2,4,4-Trimethyl-2-cyclohexen-1-yl)-2-buten-1-on], Isomuscon (Cyclohexadecanon), Jacinthaflor (2-Methyl-4-phenyl-1,3-dioxolan), Ketamber (Dodecahydro-3,8,8,11a-tetramethyl-5H-3,5a-epoxynaphth[2.1-c]oxepin), Lactojasmon (4-Hexyl-4-methyl-butyrolacton), Leguminal (Propanal-methyl-cis-3-hexenyl-acetal), Macrolide (Oxacyclohexadecan-2-on), Madranol (Mischung verschiedener Hexahydro methylionone), Magnolan (2,4-Dimethyl-5,6-indeno-1,3-dioxan), Majantol [2,2-Dimethyl-3-(3-methylphenyl)-propanol], Mandaril (3,12-Tridecadiennitril), Menthylacetat, Methylbutyrat, Methyldihydrojasmonat, Methylisobutyrat, Mintonat (3,3,5-Trimethylcyclohexylacetat), Mugetanol [1-(4-Isopropylcyclohexyl)-ethanol], Nerolione [1-(3-Methyl-2-benzofuranyl)-ethanon], Octylacetat, Ozonil (2-Tridecennitril), Palisandal (1,1-Dimethoxycyclododecan), Palisandin (Cyclododecylmethylether), Parmanyl [3-(cis-3-Hexenyloxy)-propannitril], Passifloran (3-Acetylthiohexylacetat), Peacholide (cis- und trans-3-Methyl-gamma-decalacton), Prenylsalicylat, Profarnesal (2,6,10-Trimethyl-5,9-undecadienal), Projasmon P (2-Heptylcyclopentanon), Pyroprunat (But-2-ensäurebicyclopenten-2-yl-ester), Rholiate (Kohlensäure-ethyl-2,3,6-trimethylcyclohexylester), Rosaphen (2-Methyl-5-phenylpentan-1-ol), Rosenoxid, Sandel 80 (trans-3-Isocamphylcyclohexanol), Sandranol (2-Ethyl-4-(2,2,3-trimethyl-3-cyclpenten-1-yl)-2-buten-1-ol), Symrose (4-Isoamylcyclohexanol), Symroxane (4-(3-Methylbutyl)-Cyclohexanol (Z)), Tabanon [4-(2-Butenyliden)-3,4,5-trimethyl-2-cyclohexen-1-on], Terpineol-4, Timberol (2,2,6-Trimethyl-alpha-propyl-cyclohexanpropanol), Tolylacetataldeyd D para (4-Methyl-Benzeneacetaldehyd), Tricyclodecenylpropionat, Tropicol (2-Mercapto-2-methyl-pentan-1-ol), Vertosine [2-(2,4-(oder 3,5)-Dimethyl-3-cyclohexen-1-yl)-methylenaminobenzoesäuremethylester], Vertral (Octahydro-4,7-methano-1H-indencarbaldehyd), Vetikolacetat (1,3-Dimethyl-3-phenylbutylacetat), Vetival (4-Cyclohexyl-4-methylpentan-2-on), Ysamber K (Spiro hexahydro-1',1',5',5'-tetramethyl-[1,3-dioxolan-2,8'-(5'H)-[2H-2,4a]-methanonaphthalin]. Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan; der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol; der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2;6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen; der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on; der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol; der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadiensäurenitril; 3,7-Dimethyl-6-octensäurenitril; der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat; 4-Methyl-2-pentyl-crotonat; der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate; der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal; der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate; der cyclischen Terpenaldehyde und -ketone wie z.B. Menthon; Isomenthon; 8-Mercaptomenthan-3-on; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; 2-Methyl-4-(2,6,6-trimethyl-1-cyclohexen-1-yl)-2-butenal; Nootkaton; Dihydronootkaton; 4,6,8-Megastigmatrien-3-on; alpha-Sinensal; beta-Sinensal; acetyliertes Cedernholzöl (Methylcedrylketon); der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol; der cycloaliphatischen Alkohole wie z.B. alpha,3,3-Trimethylcyclohexylmethanol; 1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol; der cyclischen und cycloaliphatischen Ether wie z.B. Cineol; Cedrylmethylether; Cyclododecylmethylether; 1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan; der cyclischen und makrocyclischen Ketone wie z. B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon; der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd; der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton; der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat; 2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat; der Ester cycloaliphatischer Alkohole wie z.B. 1-Cyclohexylethylcrotonat; der Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat; der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol; der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat; der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin; der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal; der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; 2-Benzofuranylethanon; (3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon; der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat; der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; I; Methylanthranilat; Methy-N-methylanthranilat; Schiffsche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin; 2-(3-Phenylpropyl)pyridin; Indol; Skatol; 2-Methoxy-3-isopropylpyrazin; 2-Isobutyl-3-methoxypyrazin; der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat; der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on; der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Der bzw. ein, mehrere oder sämtliche in einer erfindungsgemäßen ethanolfreien Parfümölmikroemulsion enthaltenen Riechstoffe können auch in Form von Extrakten aus natürlichen Rohstoffen, wie zum Beispiel in Form von etherischen Ölen, Concretes, Absolues, Resine, Resinoide, Balsame oder Tinkturen, in einer erfindungsgemäßen Parfümölmikroemulsion enthalten sein, vorzugsweise solchen aus der Gruppe bestehend aus: Ambratinktur; Amyrisöl; Angelicasamenöl; Angelicawurzelöl; Anisöl; Baldrianöl; Basilikumöl; Baummoos-Absolue; Bayöl; Beifussöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limetteöl destilliert; Limetteöl gepresst; Linaloeöl; Litsea-cubeba-01; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnussöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Teebaum-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen.

Der Anteil an Wasser in einer erfindungsgemäßen ethanolfreien Parfümölmikroemulsion liegt vorzugweise im Bereich von 30 - 90 Gew.-%, bevorzugt im Bereich von 40 - 70 Gew.-%, besonders bevorzugt im Bereich von 45 - 55 Gew.-%, bezogen auf das Gesamtgewicht der Parfümölmikroemulsion.

Eine erfindungsgemäße ethanolfreie Parfümölmikroemulsion umfasst vorzugsweise ein oder mehrere vicinale Diole (b) in einer Gesamtmenge von 5-50 Gew.-%, bevorzugt 10-40 Gew.-%, besonders bevorzugt 25 - 35 Gew.-%, bezogen auf das Gesamtgewicht der Parfümölmikroemulsion.

Die Gesamtmenge an Lösungsmitteln (c), insbesondere die Gesamtmenge an Verbindungen der Formeln (1) und (2), in einer erfindungsgemäßen Parfümölmikroemulsion beträgt vorzugsweise 1 - 20 Gew.-%, bevorzugt 2-10 Gew.-%, besonders bevorzugt 3 - 5 Gew.-%, bezogen auf das Gesamtgewicht der Parfümölmikroemulsion.

Die in einer erfindungsgemäßen Parfümölmikroemulsion enthaltene Menge an Riechstoffen (d) wird vom Fachmann je nach gewünschter Duftintensität gewählt. Vorzugsweise umfasst eine erfindungsgemäße Parfümölmikroemulsion eine Gesamtmenge an Riechstoffen (d) im Bereich von 1-50 Gew.-%, vorzugsweise im Bereich von 2-30 Gew.-%, weiter bevorzugt im Bereich von 4-10 Gew.-%, besonders bevorzugt im Bereich von 5 - 7 Gew.-%, bezogen auf das Gesamtgewicht der Parfümölmikroemulsion.

Zusammengefasst ist eine erfindungsgemäße Parfümölmikroemulsion besonders bevorzugt, wobei, jeweils bezogen auf das Gesamtgewicht der Parfümölmikroemulsion,
- die Gesamtmenge an Wasser (a) in der Parfümölmikroemulsion 30 - 90 Gew.-%, bevorzugt 40 - 70 Gew.-%, besonders bevorzugt 45 - 55 Gew.-%, beträgt
   und/oder
- die Gesamtmenge an vicinalen Diolen (b) in der Parfümölmikroemulsion 5-50 Gew.-%, bevorzugt 10-40 Gew.-%, besonders bevorzugt 25 - 35 Gew.-%, beträgt
- die Gesamtmenge an Riechstoffen (d) in der Parfümölmikroemulsion 1-50 Gew.-%, bevorzugt 2-30 Gew.-%, weiter bevorzugt 4-10 Gew.-%, besonders bevorzugt 5 - 7Gew.-%, beträgt.

Wie vorangehend erwähnt, führen die Lösungsmittel (c), insbesondere die Verbindungen der Formeln (1) und (2), vorteilhafterweise nicht zur Trübung einer erfindungsgemäßen Parfümölmikroemulsion. Eine erfindungsgemäße Parfümölmikroemulsion besitzt vorzugsweise einen nephelometrischen Trübungswert (NTU) von weniger als 6.

Eine erfindungsgemäße, ethanolfreie Parfümölmikroemulsion umfasst, wie vorangehend beschrieben, vorzugsweise einen oder mehrere weitere Substanzen (e). Diese sind vorzugsweise ausgewählt aus der Gruppe bestehend aus Stoffen, die Aussehen und/oder Viskosität der Parfümölmikroemulsion verändern, wie zum Beispiel Entschäumer, Verdickungsmittel oder gegebenenfalls Farbstoffe, Stoffe, die die Parfümölmikroemulsion schützen, wie zum Beispiel Konservierungsmittel, Komplexbildner, Stabilisatoren, Antioxidantien oder Lichtschutzfilter, und Stoffe, die für den Anwender einen zusätzlichen Nutzen haben, wie zum Beispiel kosmetische Wirkstoffe.

Ein weiterer Aspekt der vorliegenden Erfindung betrifft die Verwendung von Glycerol, Isobuttersäure-1-hydroxy-2,2,4-trimethyl-3-pentylester (Verbindung der Formel (1)) oder Isobuttersäure-3-hydroxy-2,2,4-trimethyl-1-pentylester (Verbindung der Formel (2)) oder einer Mischung umfassend oder bestehend aus Glycerol, Isobuttersäure-1-hydroxy-2,2,4-trimethyl-3-pentylester (Verbindung der Formel (1)) und/oder Isobuttersäure-3-hydroxy-2,2,4-trimethyl-1-pentylester (Verbindung der Formel (2)) zur Reduzierung der Klebrigkeit einer ethanolfreien Parfümölmikroemulsion.

Besonders bevorzugt ist eine erfindungsgemäße Verwendung wie vorangehend beschrieben, wobei die Parfümölmikroemulsion ein oder mehrere vicinale/s Diol(e) umfasst, wobei das bzw. eines, mehrere oder sämtliche der vicinalen Diole vorzugsweise ausgewählt ist bzw. sind aus der Gruppe bestehend aus vicinalen Diolen mit 5 bis 8 Kohlenstoff-Atomen, vorzugsweise aus der Gruppe bestehend aus 1,2-Pentandiol, 1,2-Hexandiol, 1,2-Heptandiol und 1,2-Oktandiol.

Weiter bevorzugt ist eine erfindungsgemäße Verwendung wie oben beschrieben, wobei das bzw. eines der vicinalen Diole 1,2-Hexandiol ist. Insbesondere für den Fall, dass die Parfümölmikroemulsion genau ein vicinales Diol enthält, ist dieses vorzugsweise 1,2-Hexandiol. Sofern die Parfümölmikroemulsion zwei oder mehrere vicinale Diole umfasst, handelt es sich dabei vorzugsweise um 1,2-Pentandiol und 1,2-Oktandiol. Besonders bevorzugt umfasst die Parfümölmikroemulsion 1,2-Pentandiol und 1,2-Oktandiol sowie gegebenenfalls ein oder mehrere weitere Diole, wobei das Verhältnis der Gesamtmenge an 1,2-Pentandiol zu 1,2-Oktandiol in der Parfümölmikroemulsion, bezogen auf das Gewicht, vorzugsweise im Bereich von 6 zu 1 bis 2 zu 1, bevorzugt im Bereich von 5 zu 1 bis 3 zu 1, liegt und besonders bevorzugt etwa 4 zu 1 beträgt.

Besonders bevorzugt ist eine erfindungsgemäße Verwendung, vorzugsweise eine Verwendung wie vorangehend als bevorzugt beschrieben, wobei eine Mischung umfassend oder bestehend aus einer Verbindung der Formel (1) und einer Verbindung der Formel (2) eingesetzt wird, wobei das Verhältnis der Gesamtmenge an Verbindung der Formel (1) zur Gesamtmenge an Verbindung der Formel (2) in der Mischung, bezogen auf das Gewicht, vorzugsweise im Bereich von 99 : 1 bis 1 : 99, bevorzugt im Bereich von 70 : 30 bis 30 : 70, vorzugsweise im Bereich von 60 : 40 bis 40 : 60, liegt und besonders bevorzugt etwa 44 :56 beträgt.

Ein weiterer Aspekt der vorliegenden Erfindung betrifft ein Verfahren zum Reduzieren der Klebrigkeit einer ethanolfreien Parfümölmikroemulsion, umfassend folgende Schritte:
- Bereitstellen einer ethanolfreien Parfümölmikroemulsion und
- Hinzufügen von Glycerol, Isobuttersäure-1-hydroxy-2,2,4-trimethyl-3-pentylester (Verbindung der Formel (1)) oder Isobuttersäure-3-hydroxy-2,2,4-trimethyl-1-pentylester (Verbindung der Formel (2)) oder einer Mischung umfassend oder bestehend aus Glycerol, Isobuttersäure-1-hydroxy-2,2,4-trimethyl-3-pentylester (Verbindung der Formel (1)) und/oder Isobuttersäure-3-hydroxy-2,2,4-trimethyl-1-pentylester (Verbindung der Formel (2)) zu der Parfümölmikroemulsion in einer Menge, die ausreicht, um die Klebrigkeit der Parfümölmikroemulsion (signifikant) zu reduzieren.

Nach dem Hinzufügen von Glycerol, einer Verbindung der Formel (1) und/oder einer Verbindung der Formel (2) bzw. einer entsprechenden Mischung, wie vorangehend beschrieben, entsteht vorzugsweise eine erfindungsgemäße ethanolfreie Parfümölmikroemulsion wie oben beschrieben, besonders bevorzugt eine vorangehend als bevorzugt bezeichnete, erfindungsgemäße ethanolfreie Parfümölmikroemulsion. Demnach gilt für die Bestandteile einer solchen Parfümölmikroemulsion, insbesondere für die Mengenangaben bzw. -verhältnisse der jeweiligen Bestandteile, das vorangehend Gesagte entsprechend.

Bevorzugt ist auch ein erfindungsgemäßes Verfahren wobei die bereitgestellte Parfümölmikroemulsion ein oder mehrere vicinale/s Diol(e) umfasst.
Besonders bevorzugt ist ein erfindungsgemäßes Verfahren, wie vorangehend beschrieben, wobei das bzw. eines, mehrere oder sämtliche der vicinalen Diole ausgewählt ist bzw. sind aus der Gruppe bestehend aus vicinalen Diolen mit 5 bis 8 Kohlenstoff-Atomen, vorzugsweise aus der Gruppe bestehend aus 1,2-Pentandiol, 1,2-Hexandiol, 1,2-Heptandiol und 1,2-Oktandiol. Im Übrigen gilt für die Auswahl der vicinalen Diole das vorangehend für die vicinalen Diole (b) Gesagte entsprechend.

Besonders bevorzugt ist ein erfindungsgemäßes Verfahren wie oben beschrieben, wobei eine Mischung umfassend oder bestehend aus Isobuttersäure-1-hydroxy-2,2,4-trimethyl-3-pentylester (Verbindung der Formel (1)) und Isobuttersäure-3-hydroxy-2,2,4-trimethyl-1-pentylester (Verbindung der Formel (2)) zu der Parfümölmikroemulsion hinzugefügt wird. Vorzugsweise beträgt das Verhältnis der Gesamtmenge an Isobuttersäure-1-hydroxy-2,2,4-trimethyl-3-pentylester zu Isobuttersäure-3-hydroxy-2,2,4-trimethyl-1-pentylester in der Mischung, bezogen auf das Gewicht, dabei 99 : 1 bis 1 : 99, weiter bevorzugt 70 : 30 bis 30 : 70, vorzugsweise 60 : 40 bis 40 : 60. Besonders bevorzugt beträgt das Verhältnis der Gesamtmenge an Isobuttersäure-1-hydroxy-2,2,4-trimethyl-3-pentylester zu Isobuttersäure-3-hydroxy-2,2,4-trimethyl-1-pentylester in der Mischung, bezogen auf das Gewicht, etwa 44 : 56.

Ein weiterer Aspekt der vorliegenden Erfindung betrifft einen Artikel umfassend oder bestehend aus einer erfindungsgemäßen ethanolfreien Parfümölmikroemulsion wie vorangehend beschrieben, vorzugsweise einer vorangehend als bevorzugt bezeichneten, erfindungsgemäßen Parfümölmikroemulsion.

Vorzugsweise ist ein erfindungsgemäßer Artikel ausgewählt aus der Gruppe bestehend aus Eau de Toilettes, Deodorants, Lufterfrischer, Raum- und Wäschesprays, vorzugsweise aus der Gruppe bestehend aus Eau de Toilettes, Raum- und Wäschesprays. Bevorzugt liegt ein erfindungsgemäßer Artikel nicht als Creme, Deo- oder Wachsstift vor.

Eine erfindungsgemäße Parfümölmikroemulsion kann vorteilhafterweise hergestellt werden, indem die Bestandteile der Mikroemulsion, insbesondere die Bestandteile (a) bis (e), wie jeweils vorangehend beschrieben, in beliebiger Reihenfolge (oder gleichzeitig) zusammengegeben werden. Je nach einzusetzendem Bestandteil können jedoch bestimmte, dem Fachmann bekannte und übliche Maßnahmen erforderlich sein. So ist beispielsweise 1,2-Oktandiol (als vicinales Diol (b)) vorzugsweise vor Zugabe zu den weiteren Bestandteilen der Mikroemulsion zu erwärmen, um ein besseres Lösen zu ermöglichen.

### Beispiele:

### Testbeispiel:

15 Probanden nahmen an einem Paneltest teil, um unterschiedliche Parfümölzusammensetzungen zu bewerten. Dabei wurden folgende Kriterien abgefragt:
- Klebrigkeit auf der Haut von 0 (nicht klebrig) bis 3 (sehr klebrig) und
- Austrocknend auf der Haut von 0 (nicht austrocknend) bis 3 (sehr austrocknend).

Folgende vier Parfümölzusammensetzungen wurden von jedem der Probanden bewertet:
A) Eau de Toilette (EdT): 5 Gew.-% Parfümöl 'Bioman" (vgl. Anwendungsbeispiele 1 bis 3), 95 Gew.-% 80%-iger Ethanol
B) EdT: 5 Gew.-% Parfümöl "Bioman" (vgl. Anwendungsbeispiele 1 bis 3), 28 Gew.-% 1,2-Pentandiol, 7 Gew.-% 1,2-Octylenglycol, 60 Gew.-% Wasser
C) EdT: Zusammensetzung gemäß Anwendungsbeispiel 1(s. unten)
D) EdT: Zusammensetzung gemäß Anwendungsbeispiel 2 (s. unten)

Ergebnis:

| | Klebrigkeit | Austrocknung |
|---|---|---|
| Zusammensetzung | (0-3) | (0-3) |
| A) | 0,26 | 1,74 |
| B) | 0,84 | 0,45 |
| C) | 0,67 | 0,40 |
| D) | 0,47 | 0,25 |

Wie den Ergebnissen zu entnehmen ist, resultiert die Zugabe der vicinalen Diole (siehe ethanolfreie Zusammensetzung B)) gegenüber der Ethanol-haltigen Zusammensetzung A) in erhöhter Klebrigkeit (0,84) bei reduzierten austrocknenden Eigenschaften (0,45).

Durch die in einer erfindungsgemäßen Parfümölmikroemulsion enthaltenen Lösungsmittel (c) (siehe ethanolfreie Zusammensetzungen C) und D)) kann bei weiter reduzierten austrocknenden Eigenschaften (0,40 bzw. 0,25) vorteilhafterweise eine gegenüber Zusammensetzung B) reduzierte Klebrigkeit (0,67 bzw. 0,47) erreicht werden.

### Anwendungsbeispiele:

### Anwendungsbeispiel 1 (ethanolfreie Parfümölmikroemulsion):

| Inhaltsstoff | Bezeichnung | Gew.-% |
|---|---|---|
| Bioman | Parfümöl* (Symrise) | 5,0 |
| Hydrolite-5 | 1,2-Pentandiol | 28,0 |
| Hydrolite-8 | 1,2-Octandiol | 7,0 |
| Wasser | Wasser | 50,0 |
| Glycerol | Glycerol | 10,0 |
| Summe | | 100,0 |

| | | |
|---|---|---|
| * enthaltend mindestens 92 Gew.-% Riechstoff(e) | | |

### Anwendungsbeispiel 2 (ethanolfreie Parfümölmikroemulsion):

| Inhaltsstoff | Bezeichnung | Gew.-% |
|---|---|---|
| Bioman | Parfümöl (Symrise) | 5,0 |
| Hydrolite-5 | 1,2-Pentandiol | 28,0 |
| Hydrolite-8 | 1,2-Octandiol | 7,0 |
| Wasser | Wasser | 50,0 |
| Symfresh NX | ¹⁾ | 10,0 |
| Summe | | 100,0 |

| | | |
|---|---|---|
| ¹⁾ bestehend aus der Verbindung der Formel (1) und der Verbindung der Formel (2), wie jeweils oben beschrieben, wobei das Verhältnis der Gesamtmenge an Verbindung der Formel (1) zur Gesamtmenge an Verbindung der Formel (2), bezogen auf das Gewicht, etwa 44 : 56 beträgt. | | |

### Anwendungsbeispiel 3 (ethanolfreie Parfümölmikroemulsion):

| Inhaltsstoff | Bezeichnung | Gew.-% |
|---|---|---|
| Bioman | Parfümöl (Symrise) | 5,0 |
| Hydrolite-5 | 1,2-Pentandiol | 29,0 |
| Hydrolite-8 | 1,2-Octandiol | 6,0 |
| Wasser | Wasser | 55,0 |
| Symfresh NX | (s. Anwendungsbeispiel 2) | 5,0 |
| Summe | | 100,0 |

### Anwendungsbeispiel 4 (ethanolfreie Parfümölmikroemulsion):

| Inhaltsstoff | Bezeichnung | Gew.-% |
|---|---|---|
| Open Seas | Parfümöl* (Symrise) | 7,0 |
| Hyd rolite-5 | 1,2-Pentandiol | 22,0 |
| Hydrolite-8 | 1,2-Octandiol | 5,5 |
| Wasser | Wasser | 58,0 |
| Glycerol | Glycerol | 7,5 |
| Summe | | 100,0 |

| | | |
|---|---|---|
| * enthaltend mindestens 96 Gew.-% Riechstoff(e) | | |

## Patentansprüche

1. Ethanolfreie Parfümölmikroemulsion, umfassend oder bestehend aus
(a) Wasser,
(b) einem oder mehreren vicinalen Diol(en),
(c) einem, zwei oder drei Lösungsmittel(n) zur Reduktion der Klebrigkeit, ausgewählt aus der Gruppe bestehend aus Glycerol, Isobuttersäure-1-hydroxy-2,2,4-trimethyl-3-pentylester und Isobuttersäure-3-hydroxy-2,2,4-trimethyl-1-pentylester,
(d) einem oder mehreren Riechstoff(en), und
(e) optional einer oder mehreren weiteren Substanzen,
wobei, bezogen auf das Gesamtgewicht der Parfümölmikroemulsion, die Gesamtmenge an Lösungsmitteln (c) in der Parfümölmikroemulsion 1 bis 20 Gew.-%, bevorzugt 2 bis 10 Gew.-%, besonders bevorzugt 3 bis 5 Gew.-%, beträgt.

2. Ethanolfreie Parfümölmikroemulsion nach Anspruch 1, wobei die Parfümölmikroemulsion eine Gesamtmenge an Lösungsmitteln (c) umfasst, die ausreicht, um im Vergleich zu einer identischen Parfümölmikroemulsion ohne Lösungsmittel (c) die Klebrigkeit der Parfümölmikroemulsion zu reduzieren.

3. Ethanolfreie Parfümölmikroemulsion nach Anspruch 1 oder 2, wobei das bzw. eines, mehrere oder sämtliche der vicinalen Diole (b) ausgewählt ist bzw. sind aus der Gruppe bestehend aus vicinalen Diolen mit 5 bis 8 Kohlenstoff-Atomen, vorzugsweise aus der Gruppe bestehend aus 1,2-Pentandiol, 1,2-Hexandiol, 1,2-Heptandiol und 1,2-Oktandiol.

4. Ethanolfreie Parfümölmikroemulsion nach einem der vorangehenden Ansprüche, wobei
- das bzw. eines der vicinalen Diole (b) 1,2-Hexandiol ist
oder
- die Parfümölmikroemulsion zwei oder mehrere vicinale Diole (b) umfasst und die bzw. zwei der vicinalen Diole (b) 1,2-Pentandiol und 1,2-Oktandiol sind,
wobei das Verhältnis der Gesamtmenge an 1,2-Pentandiol zu 1,2-Oktandiol in der Parfümölmikroemulsion, bezogen auf das Gewicht, vorzugsweise im Bereich von 6 : 1 bis 2 : 1, bevorzugt im Bereich von 5 : 1 bis 3 : 1, liegt und besonders bevorzugt 4 : 1 beträgt.

5. Ethanolfreie Parfümölmikroemulsion nach einem der vorangehenden Ansprüche, wobei die bzw. zwei der Lösungsmittel (c) Isobuttersäure-1-hydroxy-2,2,4-trimethyl-3-pentylester und Isobuttersäure-3-hydroxy-2,2,4-trimethyl-1-pentylester sind,
wobei das Verhältnis der Gesamtmenge an Isobuttersäure-1-hydroxy-2,2,4-trimethyl-3-pentylester zu Isobuttersäure-3-hydroxy-2,2,4-trimethyl-1-pentylester in der Parfümölmikroemulsion, bezogen auf das Gewicht, vorzugsweise im Bereich von 99 : 1 bis 1 : 99, bevorzugt im Bereich von 70 : 30 bis 30 : 70, vorzugsweise im Bereich von 60 : 40 bis 40 : 60, liegt und besonders bevorzugt 44 : 56 beträgt.

6. Ethanolfreie Parfümölmikroemulsion nach einem der vorangehenden Ansprüche, wobei, jeweils bezogen auf das Gesamtgewicht der Parfümölmikroemulsion,
- die Gesamtmenge an Wasser (a) in der Parfümölmikroemulsion 30 bis 90 Gew.-%, bevorzugt 40 bis 70 Gew.-%, besonders bevorzugt 45 bis 55 Gew.-%, beträgt
und/oder
- die Gesamtmenge an vicinalen Diolen (b) in der Parfümölmikroemulsion 5 bis 50 Gew.-%, bevorzugt 10 bis 40 Gew.-%, besonders bevorzugt 25 bis 35 Gew.-%, beträgt und/oder
- die Gesamtmenge an Riechstoffen (d) in der Parfümölmikroemulsion 1 bis 50 Gew.-%, bevorzugt 2 bis 30 Gew.-%, weiter bevorzugt 4 bis 10 Gew.-%, besonders bevorzugt 5 bis 7 Gew.-%, beträgt.

7. Verwendung von
- Glycerol, Isobuttersäure-1-hydroxy-2,2,4-trimethyl-3-pentylester oder Isobuttersäure-3-hydroxy-2,2,4-trimethyl-1-pentylester
oder
- einer Mischung umfassend oder bestehend aus Glycerol, Isobuttersäure-1-hydroxy-2,2,4-trimethyl-3-pentylester und/oder Isobuttersäure-3-hydroxy-2,2,4-trimethyl-1-pentylester
zur Reduzierung der Klebrigkeit einer ethanolfreien Parfümölmikroemulsion.

8. Verwendung nach Anspruch 7, wobei die Parfümölmikroemulsion ein oder mehrere vicinale/s Diol(e) umfasst, wobei das bzw. eines, mehrere oder sämtliche der vicinalen Diole vorzugsweise ausgewählt ist bzw. sind aus der Gruppe bestehend aus vicinalen Diolen mit 5 bis 8 Kohlenstoff-Atomen, vorzugsweise aus der Gruppe bestehend aus 1,2-Pentandiol, 1,2-Hexandiol, 1,2-Heptandiol und 1,2-Oktandiol.

9. Verwendung nach Anspruch 7 oder 8, wobei
- das bzw. eines der vicinalen Diole 1,2-Hexandiol ist
oder
- die Parfümölmikroemulsion zwei oder mehrere vicinale Diole umfasst und die bzw. zwei der vicinalen Diole 1,2-Pentandiol und 1,2-Oktandiol sind,
wobei das Verhältnis der Gesamtmenge an 1,2-Pentandiol zu 1,2-Oktandiol in der Parfümölmikroemulsion, bezogen auf das Gewicht, vorzugsweise im Bereich von 6 : 1 bis 2 : 1, bevorzugt im Bereich von 5 : 1 bis 3 : 1, liegt und besonders bevorzugt 4 : 1 beträgt.

10. Verwendung einer Mischung, umfassend oder bestehend aus Isobuttersäure-1-hydroxy-2,2,4-trimethyl-3-pentylester und Isobuttersäure-3-hydroxy-2,2,4-trimethyl-1-pentylester, nach einem der Ansprüche 7 bis 9, wobei das Verhältnis der Gesamtmenge an Isobuttersäure-1-hydroxy-2,2,4-trimethyl-3-pentylester zu Isobuttersäure-3-hydroxy-2,2,4-trimethyl-1-pentylester in der Mischung, bezogen auf das Gewicht, vorzugsweise im Bereich von 99 : 1 bis 1 : 99, bevorzugt im Bereich von 70 : 30 bis 30 : 70, vorzugsweise im Bereich von 60 : 40 bis 40 : 60, liegt und besonders bevorzugt etwa 44 : 56 beträgt.

11. Verfahren zum Reduzieren der Klebrigkeit einer ethanolfreien Parfümölmikroemulsion, umfassend folgende Schritte:
- Bereitstellen einer ethanolfreien Parfümölmikroemulsion und
- Hinzufügen von Glycerol, Isobuttersäure-1-hydroxy-2,2,4-trimethyl-3-pentylester oder Isobuttersäure-3-hydroxy-2,2,4-trimethyl-1-pentylester oder einer Mischung umfassend oder bestehend aus Glycerol, Isobuttersäure-1-hydroxy-2,2,4-trimethyl-3-pentylester und/oder Isobuttersäure-3-hydroxy-2,2,4-trimethyl-1-pentylester zu der Parfümölmikroemulsion in einer Menge, die ausreicht, um die Klebrigkeit der Parfümölmikroemulsion zu reduzieren.

12. Verfahren nach Anspruch 11, wobei die bereitgestellte Parfümölmikroemulsion ein oder mehrere vicinale/s Diol(e) umfasst,
wobei das bzw. eines, mehrere oder sämtliche der vicinalen Diole vorzugsweise ausgewählt ist bzw. sind aus der Gruppe bestehend aus vicinalen Diolen mit 5 bis 8 Kohlenstoff-Atomen, vorzugsweise aus der Gruppe bestehend aus 1,2-Pentandiol, 1,2-Hexandiol, 1,2-Heptandiol und 1,2-Oktandiol.

13. Verfahren nach Anspruch 11 oder 12, wobei eine Mischung umfassend oder bestehend aus Isobuttersäure-1-hydroxy-2,2,4-trimethyl-3-pentylester und Isobuttersäure-3-hydroxy-2,2,4-trimethyl-1-pentylester zu der Parfümölmikroemulsion hinzugefügt wird, und wobei das Verhältnis der Gesamtmenge an Isobuttersäure-1-hydroxy-2,2,4-trimethyl-3-pentylester zu Isobuttersäure-3-hydroxy-2,2,4-trimethyl-1-pentylester in der Mischung, bezogen auf das Gewicht, vorzugsweise im Bereich von 99 : 1 bis 1 : 99, bevorzugt im Bereich von 70 : 30 bis 30 : 70, vorzugsweise im Bereich von 60 : 40 bis 40 : 60, liegt und besonders bevorzugt etwa 44 : 56 beträgt.

14. Artikel umfassend eine ethanolfreie Parfümölmikroemulsion nach einem der Ansprüche 1 bis 6.

15. Artikel nach Anspruch 14, wobei der Artikel ausgewählt ist aus der Gruppe bestehend aus Eau de Toilettes und Lufterfrischer, vorzugsweise aus der Gruppe bestehend aus Eau de Toilettes, Raum- und Wäschesprays.

## Claims

1. Ethanol-free perfume oil microemulsion, comprising or consisting of
(a) water,
(b) one or more vicinal diol(s),
(c) one, two or more solvent(s) to reduce adhesiveness, selected from the group consisting of glycerol, isobutyric acid-1-hydroxy-2,2,4-trimethyl-3-pentyl ester and isobutyric acid-3-hydroxy-2,2,4-trimethyl-1-pentyl ester,
(d) one or more fragrance(s), and
(e) optionally one ore more further substances,
wherein, with respect to the total weight of the perfume oil microemulsion, the total amount of solvents (c) in the perfume oil microemulsion is 1 to 20 wt.-%, preferably 2 to 10 wt.-%, particularly preferably 3 to 5 wt.-%.

2. Ethanol-free perfume oil microemulsion according to claim 1, wherein the perfume oil microemulsion comprises a total amount of solvents (c), which is sufficient to reduce the adhesiveness of the perfume oil microemulsion compared to an identical perfume oil microemulsion without solvent (c).

3. Ethanol-free perfume oil microemulsion according to claim 1 or 2, wherein the one or one of, or several or all of the vicinal diols (b), is/are selected from the group consisting of vicinal diols with 5 to 8 carbon atoms, preferably from the group consisting of 1,2-pentane diol, 1,2-hexane diol, 1,2-heptane diol and 1,2-octane diol.

4. Ethanol-free perfume oil microemulsion according to one of the previous claims, wherein
- the one or one of the vicinal diols (b), is 1,2-hexane diol
or
- the perfume oil microemulsion comprises two or more vicinal diols (b) and the two or two of the vicinal diols (b), respectively, are 1,2-pentane diol and 1,2-octane diol,
wherein the ratio of the total amount of 1,2-pentane diol to 1,2-octane diol in the perfume oil microemulsion, with respect to the weight, is preferably in the range of 6 : 1 to 2 : 1, further preferably in the range of 5 : 1 to 3 : 1 and particularly preferably is 4 : 1.

5. Ethanol-free perfume oil microemulsion according to one of the previous claims, wherein the two or two of the solvents (c), respectively, are isobutyric acid-1-hydroxy-2,2,4-trimethyl-3-pentyl ester and isobutyric acid-3-hydroxy-2,2,4-trimethyl-1-pentyl ester,
wherein the ratio of the total amount of isobutyric acid-1-hydroxy-2,2,4-trimethyl-3-pentyl ester to isobutyric acid-3-hydroxy-2,2,4-trimethyl-1-pentyl ester in the perfume oil microemulsion, with respect to the weight, is preferably in the range of 99 : 1 to 1 : 99, further preferably in the range of 70 : 30 to 30 : 70, further preferably in the range of 60 : 40 to 40 : 60, and particularly preferably is 44 : 56.

6. Ethanol-free perfume oil microemulsion according to one of the previous claims, wherein, in each case with respect to the total weight of the perfume oil microemulsion,
- the total amount of water (a) in the perfume oil microemulsion is 30 to 90 wt.-%, preferably 40 to 70 wt.-%, particularly preferably 45 to 55 wt.-%
and/or
- the total amount of vicinal diols (b) in the perfume oil microemulsion is 5 to 50 wt.-%, preferably 10 to 40 wt.-%, particularly preferably 25 to 35 wt.-%
and/or
- the total amount of fragrances (d) in the perfume oil microemulsion is 1 to 50 wt.-%, preferably 2 to 30 wt.-%, further preferably 4 to 10 wt.-%, particularly preferably 5 to 7 wt.-%.

7. Use of
- glycerol, isobutyric acid-1-hydroxy-2,2,4-trimethyl-3-pentyl ester or isobutyric acid-3-hydroxy-2,2,4-trimethyl-1-pentyl ester
or
- a mixture comprising or consisting of glycerol, isobutyric acid-1-hydroxy-2,2,4-trimethyl-3-pentyl ester and/or isobutyric acid-3-hydroxy-2,2,4-trimethyl-1-pentyl ester
for reducing the adhesiveness of an ethanol-free perfume oil microemulsion.

8. Use according to claim 7, wherein the perfume oil microemulsion comprises one or more vicinal diol(s), wherein the one, several or all of the vicinal diols, is/are preferably selected from the group consisting of vicinal diols with 5 to 8 carbon atoms, preferably from the group consisting of 1,2-pentane diol, 1,2-hexane diol, 1,2-heptane diol and 1,2-octane diol.

9. Use according to claim 7 or 8, wherein
- the one or one of the vicinal diols, is 1,2-hexane diol
or
- the perfume oil microemulsion comprises two or more vicinal diols (b) and the two or two of the vicinal diols (b), are 1,2-pentane diol and 1,2-octane diol,
wherein the ratio of the total amount of 1,2-pentane diol to 1,2-octane diol in the perfume oil microemulsion, with respect to the weight, is preferably in the range of 6 : 1 to 2 : 1, further preferably in the range of 5 : 1 to 3 : 1 and particularly preferably is 4 : 1.

10. Use of a mixture, comprising or consisting of isobutyric acid-1-hydroxy-2,2,4-trimethyl-3-pentyl ester and isobutyric acid-3-hydroxy-2,2,4-trimethyl-1-pentyl ester, according to one of the claims 7 to 9, wherein the ratio of the total amount of isobutyric acid-1-hydroxy-2,2,4-trimethyl-3-pentyl ester to isobutyric acid-3-hydroxy-2,2,4-trimethyl-1-pentyl ester in the mixture, with respect to the weight, is preferably in the range of 99 : 1 to 1 : 99, further preferably in the range of 70 : 30 to 30 : 70, further preferably in the range of 60 : 40 to 40 : 60, and particularly preferably is 44 : 56.

11. Method for reducing the adhesiveness of an ethanol-free perfume oil microemulsion, comprising the following steps:
- providing an ethanol-free perfume oil microemulsion,
- adding of glycerol, isobutyric acid-1-hydroxy-2,2,4-trimethyl-3-pentyl ester or isobutyric acid-3-hydroxy-2,2,4-trimethyl-1-pentyl ester or of a mixture comprising or consisting of glycerol, isobutyric acid-1-hydroxy-2,2,4-trimethyl-3-pentyl ester and/or isobutyric acid-3-hydroxy-2,2,4-trimethyl-1-pentyl ester to the perfume oil microemulsion in an amount sufficient to reduce adhesiveness of the perfume oil microemulsion.

12. Method according to claim 11, wherein the provided perfume oil microemulsion comprises one or more vicinal diol(s),
wherein the one, several or all of the vicinal diols, is/are preferably selected from the group consisting of vicinal diols with 5 to 8 carbon atoms, preferably from the group consisting of 1,2-pentane diol, 1,2-hexane diol, 1,2-heptane diol and 1,2-octane diol.

13. Method according to claim 11 or 12, wherein a mixture comprising or consisting of isobutyric acid-1-hydroxy-2,2,4-trimethyl-3-pentyl ester and isobutyric acid-3-hydroxy-2,2,4-trimethyl-1-pentyl ester is added to the perfume oil microemulsion, and wherein the ratio of the total amount of isobutyric acid-1-hydroxy-2,2,4-trimethyl-3-pentyl ester to isobutyric acid-3-hydroxy-2,2,4-trimethyl-1-pentyl ester in the mixture, with respect to the weight, is preferably in the range of 99 : 1 to 1 : 99, further preferably in the range of 70 : 30 to 30 : 70, further preferably in the range of 60 : 40 to 40 : 60, and particularly preferably is 44 : 56.

14. Product comprising an ethanol-free perfume oil microemulsion according to one of the claims 1 to 6.

15. Product according to claim 14, wherein the product is selected from the group consisting of eau de toilettes and air refreshers, preferably from the group consisting of eau de toilettes, room sprays and laundry sprays.

## Revendications

1. Microémulsion d'huile de parfum exempte d'éthanol, comprenant ou se composant
(a) d'eau
(b) d'un ou de plusieurs diol(s) vicinal (vicinaux),
(c) d'un, de deux ou de trois solvant(s) de réduction de l'adhésivité, choisi(s) dans le groupe constitué par le glycérol, l'ester 1-hydroxy-2,2,4-triméthyl-3-pentylique de l'acide isobutyrique et l'ester 3-hydroxy-2,2,4-triméthyl-1-pentylique de l'acide isobutyrique,
(d) d'une ou de plusieurs substance(s) odoriférante(s) et
(e) optionnellement, d'une ou de plusieurs autre(s) substance(s),
dans laquelle la quantité totale de solvants (c) dans la microémulsion d'huile de parfum, par rapport au poids total de la microémulsion d'huile de parfum, est comprise entre 1 et 20 % en poids, de préférence entre 2 et 10 % en poids, d'une manière particulièrement préférée entre 3 et 5 % en poids.

2. Microémulsion d'huile de parfum exempte d'éthanol, selon la revendication 1, dans laquelle la microémulsion d'huile de parfum comprend une quantité totale de solvants (c) qui est suffisante pour réduire, en comparaison d'une microémulsion d'huile de parfum identique sans solvant (c), l'adhésivité de la microémulsion d'huile de parfum.

3. Microémulsion d'huile de parfum exempte d'éthanol, selon la revendication 1 ou 2, dans laquelle le diol vicinal ou bien l'un des, plusieurs des ou tous les diols vicinaux (b) est ou bien sont choisi(s) dans le groupe constitué par les diols vicinaux ayant 5 à 8 atomes de carbone, de préférence dans le groupe constitué par le pentanediol-1,2, l'hexanediol-1,2, l'heptanediol-1,2 et l'octanediol-1,2.

4. Microémulsion d'huile de parfum exempte d'éthanol, selon l'une quelconque des revendications précédentes, dans laquelle
- ledit ou bien l'un des diols vicinaux (b) est l'hexanediol-1,2
ou
- la microémulsion d'huile de parfum comprend un ou plusieurs diols vicinaux (b) et les ou bien deux des diols vicinaux (b) sont le pentanediol-1,2 et l'octanediol-1,2,
le rapport de la quantité totale du pentanediol-1,2 à l'octanediol-1,2 dans la microémulsion d'huile de parfum, par rapport au poids, étant compris, de préférence, dans la gamme allant de 6 : 1 à 2 : 1, de préférence dans la gamme allant de 5 : 1 à 3 : 1 et étant, d'une manière particulièrement préférée de 4 : 1.

5. Microémulsion d'huile de parfum exempte d'éthanol, selon l'une quelconque des revendications précédentes, dans laquelle les ou bien deux des solvants (c) sont l'ester 1-hydroxy-2,2,4-triméthyl-3-pentylique de l'acide isobutyrique et l'ester 3-hydroxy-2,2,4-triméthyl-1-pentylique de l'acide isobutyrique,
le rapport de la quantité totale de l'ester 1-hydroxy-2,2,4-triméthyl-3-pentylique de l'acide isobutyrique à l'ester 3-hydroxy-2,2,4-triméthyl-1-pentylique de l'acide isobutyrique dans la microémulsion d'huile de parfum, par rapport au poids, étant compris, de préférence, dans la gamme allant de 99 : 1 à 1 : 99, de préférence dans la gamme allant de 70 : 30 à 30 : 70, de préférence dans la gamme allant de 60 : 40 à 40 : 60 et étant, d'une manière particulièrement préférée, de 44 : 56.

6. Microémulsion d'huile de parfum exempte d'éthanol, selon l'une quelconque des revendications précédentes, dans laquelle, respectivement par rapport au poids total de la microémulsion d'huile de parfum,
- la quantité totale d'eau (a) dans la microémulsion d'huile de parfum est comprise entre 30 et 90 % en poids, de préférence entre 40 et 70 % en poids, d'une manière particulièrement préférée entre 45 et 55 % en poids,
et/ou
- la quantité totale de diols vicinaux (b) dans la microémulsion d'huile de parfum est comprise entre 5 et 50 % en poids, de préférence entre 10 et 40 % en poids, d'une manière particulièrement préférée entre 25 et 35 % en poids,
et/ou
- la quantité totale de substances odoriférantes (d) dans la microémulsion d'huile de parfum est comprise entre 1 et 50 % en poids, de préférence entre 2 et 30 % en poids, encore de préférence entre 4 et 10 % en poids, d'une manière particulièrement préférée entre 5 et 7 % en poids.

7. Utilisation
- de glycérol, de l'ester 1-hydroxy-2,2,4-triméthyl-3-pentylique de l'acide isobutyrique ou de l'ester 3-hydroxy-2,2,4-triméthyl-1-pentylique de l'acide isobutyrique
ou
- d'un mélange comprenant ou se composant de glycérol, d'ester 1-hydroxy-2,2,4-triméthyl-3-pentylique de l'acide isobutyrique et/ou d'ester 3-hydroxy-2,2,4-triméthyl-1-pentylique de l'acide isobutyrique,
afin de réduire l'adhésivité d'une microémulsion d'huile de parfum exempte d'éthanol.

8. Utilisation selon la revendication 7, dans laquelle la microémulsion d'huile de parfum comprend un ou plusieurs diol(s) vicinal (vicinaux), ledit ou bien l'un, plusieurs des ou tous les diols vicinaux étant choisi(s) de préférence dans le groupe constitué par les diols vicinaux ayant 5 à 8 atomes de carbone, de préférence dans le groupe constitué par le pentanediol-1,2, l'hexanediol-1,2, l'heptanediol-1,2 et l'octanediol-1,2.

9. Utilisation selon la revendication 7 ou 8, dans laquelle
- ledit ou bien l'un des diols vicinaux (b) est l'hexanediol-1,2
ou
- la microémulsion d'huile de parfum comprend deux ou plusieurs diols vicinaux et les ou bien deux des diols vicinaux sont le pentanediol-1,2 et l'octanediol-1,2,
le rapport de la quantité totale du pentanediol-1,2 à l'octanediol-1,2 dans la microémulsion d'huile de parfum, par rapport au poids, étant compris, de préférence, dans la gamme allant de 6 : 1 à 2 : 1, de préférence dans la gamme allant de 5 : 1 à 3 : 1 et étant, d'une manière particulièrement préférée de 4 : 1.

10. Utilisation d'un mélange, comprenant ou composé de l'ester 1-hydroxy-2,2,4-triméthyl-3-pentylique de l'acide isobutyrique et de l'ester 3-hydroxy-2,2,4-triméthyl-1-pentylique de l'acide isobutyrique, selon l'une quelconque des revendications 7 à 9, le rapport de la quantité totale de l'ester 1-hydroxy-2,2,4-triméthyl-3-pentylique de l'acide isobutyrique à l'ester 3-hydroxy-2,2,4-triméthyl-1-pentylique de l'acide isobutyrique dans le mélange, par rapport au poids, étant compris, de préférence, dans la gamme allant de 99 : 1 à 1 : 99, de préférence dans la gamme allant de 70 : 30 à 30 : 70, de préférence dans la gamme allant de 60 : 40 à 40 : 60 et étant, d'une manière particulièrement préférée, de 44 : 56.

11. Procédé de réduction de l'adhésivité d'une microémulsion d'huile de parfum exempte d'éthanol, comprenant les étapes suivantes :
- fournir une microémulsion d'huile de parfum exempte d'éthanol et
- ajouter, à la microémulsion d'huile de parfum, du glycérol, de l'ester 1-hydroxy-2,2,4-triméthyl-3-pentylique de l'acide isobutyrique ou de l'ester 3-hydroxy-2,2,4-triméthyl-1-pentylique de l'acide isobutyrique ou un mélange comprenant ou composé de glycérol, d'ester 1-hydroxy-2,2,4-triméthyl-3-pentylique de l'acide isobutyrique et/ou d'ester 3-hydroxy-2,2,4-triméthyl-1-pentylique de l'acide isobutyrique, en une quantité qui est suffisante pour réduire l'adhésivité de la microémulsion d'huile de parfum.

12. Procédé selon la revendication 11, dans lequel la microémulsion d'huile de parfum fournie comprend un ou plusieurs diol(s) vicinal (vicinaux),
dans lequel ledit ou bien l'un, plusieurs des ou tous les diols vicinaux est ou bien sont choisi(s) dans le groupe constitué par les diols vicinaux ayant 5 à 8 atomes de carbone, de préférence dans le groupe constitué par le pentanediol-1,2, l'hexanediol-1,2, l'heptanediol-1,2 et l'octanediol-1,2.

13. Procédé selon la revendication 11 ou 12, dans lequel on ajoute, à la microémulsion d'huile de parfum, un mélange comprenant ou composé d'ester 1-hydroxy-2,2,4-triméthyl-3-pentylique de l'acide isobutyrique et d'ester 3-hydroxy-2,2,4-triméthyl-1-pentylique de l'acide isobutyrique, et le rapport de la quantité totale de l'ester 1-hydroxy-2,2,4-triméthyl-3-pentylique de l'acide isobutyrique à l'ester 3-hydroxy-2,2,4-triméthyl-1-pentylique de l'acide isobutyrique dans le mélange, par rapport au poids, étant compris, de préférence, dans la gamme allant de 99 : 1 à 1 : 99, de préférence dans la gamme allant de 70 : 30 à 30 : 70, de préférence dans la gamme allant de 60 : 40 à 40 : 60 et étant, d'une manière particulièrement préférée, de 44 : 56.

14. Article comprenant une microémulsion d'huile de parfum exempte d'éthanol selon l'une quelconque des revendications 1 à 6.

15. Article selon la revendication 14, ledit article étant choisi dans le groupe constitué par les eaux de toilette et les produits rafraîchissant l'air, de préférence dans le groupe constitué par les eaux de toilette, les sprays pour locaux et linge.
